# EUROPEAN PATENT APPLICATION

(11) **EP 1 127 540 A1**
(43) Date of publication of application: **29.08.2001**
(21) Application number: 01103685.2
(22) Date of filing: 23.02.2001
(51) Int. Cl.: A61B 5/05

(54) **Portable body fat monitor**

(30) Priority: 25.02.2000 US 185176 P; 16.02.2001 US 784026
(71) Applicant: TANITA CORPORATION, Tokyo 174 (JP)
(72) Inventor: Benson, Carol A., Elgin, Illinois 60120 (US); Cage, Kenneth L., Gaithersburg, Maryland 20887 (US); Rubinson, Gene Z., Rockville, Maryland 20850 (US); Iwabuchi, Tamotsu, Itabashi-ku, Tokyo (JP)
(74) Representative: Müller-Boré & Partner Patentanwälte

(57) **Abstract**

A portable body fat monitor is provided having electrodes arranged so that the instrument is easily operated by a user, thereby facilitating accurate measurements. Embodiments include a portable body fat monitor having an insulating main body, shaped substantially like a rectangular parallelepiped. Four electrodes are spaced apart from one another on a side surface of the main body. A display unit, such as a liquid crystal display, is disposed on a front face of the main body, and an operator unit, such as a set of push buttons, is located below the display unit on the front face of the main body. To operate the monitor, a user enters numerical values representing his or her height and weight with the thumbs of their right and left hands. The middle and index fingers of the user's left hand are then brought into contact with two of the electrodes, respectively. Likewise, the index and middle fingers of the right hand are brought into contact with the other two electrodes, respectively, for the body fat measurement. The pairs of electrodes are spaced apart, for preventing the fingers of the right and left hands from touching, and the main body is sized to allow the user to easily hold the monitor.

## Description

### FIELD OF THE INVENTION

The present invention relates to a portable body fat monitor, and more particularly to measuring body impedance using four electrodes and calculating body fat occupancy ratio based on the measured electric impedance.

### BACKGROUND ART

A conventional portable body fat monitor is disclosed in U.S. Patent 4,949,727. This body fat monitor has a display and an operator unit formed on the face of a main body. Four measurement electrodes are formed in pairs on the back of the main body. Each of the measurement electrodes is touched with one finger of the right or left hand. In operation, all four back surface electrodes are pressed simultaneously to generate a display readout on the opposite front surface.

Namco Ltd. has put a body fat monitor, which looks like a pocket game machine, on the market. This monitor has an electrode formed on each opposing side of a main body.

Daiwa Sieko, Ltd. has marketed portable body fat monitors named "Poke-navi" and "Poke-mini" having electrodes formed on the upper left and right parts of the face and back of a main body.

Japanese Unexamined Patent Application Publication No. 11-700092 discloses a portable body fat monitor having electrodes formed on the top and bottom of a card.

The body fat monitor disclosed in U.S. Patent 4,949,727 suffers from poor maneuverability. The four measurement electrodes are formed on the back, and each of the electrodes must be touched with one finger of the right or left hand. Disadvantageously, the spacing between each pair of electrodes is very narrow, so precision measurement is compromised and the monitor is awkward to use.

The body fat monitor marketed by Namco Ltd. suffers from the same drawback, i.e., lack of precision, as the body fat monitor described in U.S. Patent 4,949,727. The monitor is small enough to be enclosed in one hand. When the monitor is held with both hands for measurement, there is a tendency for the fingers of the right and left hands to touch each other, leading to unacceptable error in measurement.

The body fat monitor sold by Daiwa Seiko, Ltd. also suffers from poor maneuverability. An operator switch must be pressed while the electrodes are touched with the fingers, so the fingers must be largely separated from one another or the main body must be held in an awkward manner.

The monitor disclosed in the Japanese Unexamined Patent Application Publication No. 11-70092 has the electrodes distributed at the four comers of the monitor.
Consequently, lengthy connections are required between the electrodes and the monitor's circuitry. The monitor is therefore susceptible to extraneous electric noises or the like.

There exists a need for a portable body fat monitor that provides accurate measurements and is easy to operate.

### SUMMARY OF THE INVENTION

An advantage of the present invention is a portable body fat monitor having electrodes arranged so that the instrument is easily operated by a user, thereby facilitating accurate measurements.

Additional advantages and other features of the present invention will be set forth in part in the description which follows and in part will become apparent to those having ordinary skill in the art upon examination of the following or may be learned from the practice of the invention. The advantages of the invention may be realized and obtained as particularly pointed out in the appended claims.

According to the present invention, the foregoing and other advantages are achieved in part by a portable body fat monitor comprising a main body having a front face and a side surface; four electrodes spaced apart on the side surface of the main body; a display unit on the front face of the main body; and an operator unit on the front face of the main body. The main body is substantially parallelepiped-shaped. Thus, the user can hold the monitor easily and in a stable manner, leading to increased precision in measurement.

Additional advantages of the present invention will become readily apparent to those skilled in this art from the following detailed description, wherein only the preferred embodiment of the present invention is shown and described, simply by way of illustration of the best mode contemplated for carrying out the present invention. As will be realized, the present invention is capable of other and different embodiments, and its several details are capable of modifications in various obvious respects, all without departing from the invention. Accordingly, the drawings and description are to be regarded as illustrative in nature, and not as restrictive.

### BRIEF DESCRIPTION OF THE DRAWINGS

Reference is made to the attached drawings, wherein elements having the same reference numeral designations represent like elements throughout, and wherein:
Fig. 1 illustrates a portable body fat monitor in accordance with an embodiment of the present invention;
Fig. 2 schematically illustrates the electric circuitry of the body fat monitor shown in Fig. 1;
Fig. 3 is a flowchart describing actions to be performed in the body fat monitor shown in Fig. 1.
Fig. 4 illustrates a portable body fat monitor in accordance with an embodiment of the present invention.

### DESCRIPTION OF THE INVENTION

Conventional portable body fat monitors have their components arranged such that the instruments are difficult for the user to manipulate, resulting in inaccurate measurements. The present invention addresses and solves these problems stemming from conventional non-ergonomic portable body fat monitors.

According to the present invention, a portable body fat monitor is provided having four electrodes disposed spaced apart from each other on a side surface of a main body, such as an upper or a lower side surface. A display unit, such as a liquid-crystal display, and an operator unit, such as a set of switches, are formed on a front face of the main body. Thus, extraneous electrical noise is blocked, precision of measurement is improved, and maneuverability of the device is improved.

In one embodiment of the present invention, a portable body fat monitor has an insulating main body, shaped substantially like a rectangular parallelepiped. The four electrodes are spaced apart from one another on a side surface of the main body, and the operator unit is located below the display unit on the front face of the main body. A user can therefore enter numerical values representing his or her height and weight with the thumbs of their right and left hands, as if the user were manipulating a game machine. Moreover, the distance from the operator unit to the lower side surface is so short that the monitor can be held using the thumbs, and the thumbs can be moved readily while manipulating the operator unit and holding the monitor. When operating the body fat monitor, the middle and index fingers of the user's left hand are brought into contact with two of the electrodes, respectively. Likewise, the index and middle fingers of the right hand are brought into contact with the other two electrodes, respectively, for the body fat measurement. Alternatively, any of the user's other fingers may be brought into contact with the electrodes. The pairs of electrodes are spaced apart, for preventing the fingers of the right and left hands from touching, and the main body is sized to allow the user to easily hold the body fat monitor.

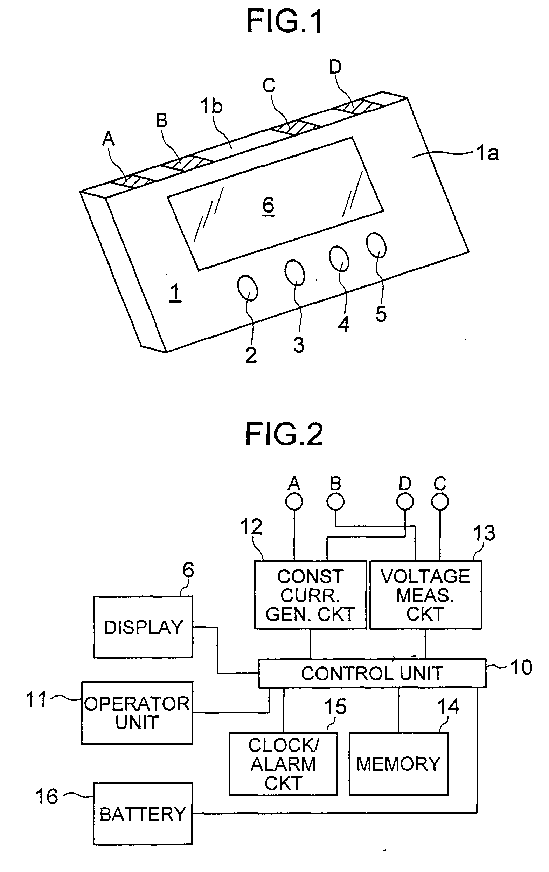

An embodiment of the present invention will now be described with reference to the drawings. Referring to Fig. 1, a Mode switch 2, such as a push button, used to switch the power supply of the monitor and select a mode, is located in the lower left portion of the front face 1a of a main body 1 of a portable body fat monitor. An Up switch 3, a Down switch 4, and an Enter switch 5, such as push buttons, are located to the right of the Mode switch 2.

When the Mode switch 2 is operated once, the power supply is turned on. The monitor is placed in a display mode in which data stored in a memory indicating the functions of the monitor is displayed. When the Mode switch 2 is operated again, the body fat measurement mode is switched to a re-measurement mode in which a body fat occupancy ratio is measured using data stored in the memory. When the Mode switch 2 is operated again, the re-measurement mode is switched to a new measurement mode in which a body fat occupancy ratio is measured anew. When the Mode switch 2 is operated again, the new measurement mode is switched to an alarm setting mode in which an alarm time is set, enabling a body fat measurement to be taken, for example, at a predetermined time every day. Every time the Mode switch 2 is operated, one mode is switched to another.

When the Up switch 3 is repeatedly operated or held on in the new measurement mode or alarm setting mode, the value of displayed data is increased. The Down switch 4 is used to decrease the displayed data that has been increased using the Up switch 3. The Enter switch 5 is used to enter the displayed data, which has'been set using the Up switch 3 and Down switch 4 in each of the foregoing modes, as a finalized value into a control unit 10 or a memory 14 shown in Fig. 2. A display unit 6 is disposed on the upper part of front face 1a of the main body 1 in order to display data that is set or entered using the switches, the results of measurement, prompts for the user, the time, etc.

An electrode A having an inductive metallic chip or a metallic plating is located near the left edge of an upper side surface 1b of the main body 1. Electrodes B, C, and D made of the same material as the electrode A are disposed to the right of electrode A on side surface 1b and are spaced apart from each other and from electrode A. In other words, the four electrodes A-D are arranged in a row on side surface 1b. When operating the body fat monitor, the middle and index fingers of the user's left hand are brought into contact with the electrodes A and B respectively. Likewise, the index and middle fingers of the right hand are brought into contact with the electrodes C and D respectively. Alternatively, any of the user's other fingers may be brought into contact with the electrodes. The pairs of electrodes A,B and C,D are spaced apart, for preventing the fingers of the right and left hands from touching.

Fig. 2 shows the electric circuitry of the portable body fat monitor shown in Fig. 1. A control unit 10 processes data sent from the operator unit 11 composed of the Mode switch 2, Up switch 3, Down switch 4 and Enter switch 5. The results of arithmetic operations are displayed on the display unit 6. A constant current generation circuit 12 for supplying a constant current to the electrodes A and D is connected to the control unit 10. A voltage measurement circuit 13 for measuring the voltages at the electrodes B and C is also connected to the control circuit 10. A memory 14 for storing data read at the operator unit 11 and data indicating the results of arithmetic operations is also connected to the control unit 10. A conventional clock and alarm circuit 15 for indicating a current time or an alarm time on the display unit 6 is connected to the control unit 10.

When the Mode switch 2 is operated, electricity is supplied from a battery 16 to the components. An automatic power off timer is incorporated in the control unit 10 in order to turn off the supply of electricity.

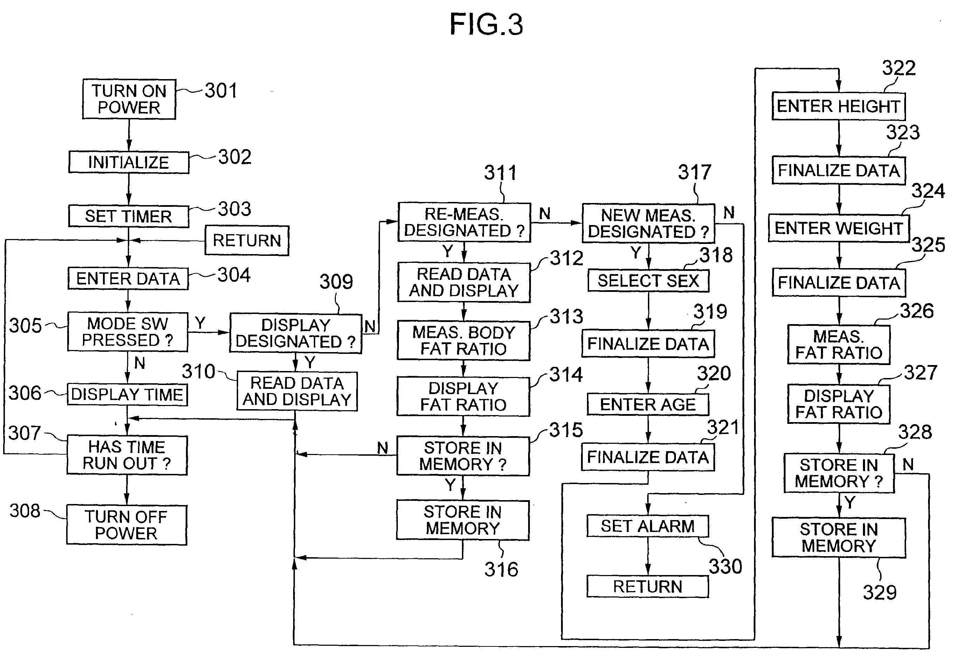

Fig. 3 is a flowchart describing actions to be performed in an embodiment of the present invention. Using the flowchart, the actions to be performed in a portable body fat monitor according to the present invention will be described. First, the Mode switch 2 shown in Fig. 1 is operated to turn on the monitor. Electricity is then supplied to the circuits and control unit shown in Fig. 2 (step 301). At step 302, the display unit and circuits are initialized. At step 303, the automatic power off timer incorporated in the control unit 10 is set. When none of the switches 2, 3, 4, 5 has been pressed for a predetermined time, for example, five minutes, the timer indicates a time-out (step 307). At step 308, the power supply is turned off in order to prevent exhaustion of the battery 16 from occurring when the instrument is neglected.

At step 304, on/off-state data sent from the switches is fetched into the control unit 10. Control is then passed to step 305. At step 305, it is judged whether data was entered using the Mode switch 2. When the power supply is turned on, control is passed to step 306. A current time is then indicated on the display unit 6 according to time data sent from the clock and alarm circuit 15, using well-known techniques. A description of the time indication processing will therefore be omitted herein. At step 307, judgment is made in the negative unless the predetermined time elapses. Control is then returned to step 304, and the current time indication is continued. Data entered using switches 2-5 is then repeatedly awaited.

If the Mode switch 2 is operated while data entry is awaited, control is passed from step 305 to the display mode involving steps 309 and 310. When the Mode switch 2 is operated once, the indication of the current time is deleted, and at step 310, data including a user's sex, age, height, and body fat occupancy ratio, which have been entered or measured previously, is read from the memory 14 and indicated on the display unit 6. This indication is displayed continuously unless the time runs out at step 307 or the Mode switch 2 is operated again.

In the display mode, when the Mode switch 2 is operated, judgment is made in the negative at step 309. Control is then passed to the re-measurement mode involving step 311. When the re-measurement mode is established, the indication displayed in the display mode is deleted. At step 312, data including the user's sex, age, height, and weight is read from the memory 14 and indicated on the display unit 6. A message saying that a body fat occupancy ratio will be calculated based on the data is then indicated on the display unit 6. At step 313, a constant current flows from the constant current generation circuit 12 shown in Fig. 2 to the middle fingers of the right and left hands of a user through the electrodes A and D. The voltage measurement circuit 13 measures voltages developed at the index fingers of the user's right and left hands through the electrodes B and C. An electric impedance occurring between the user's index fingers is calculated according to an expression of "resistance = voltage ÷ current." Based on the electric impedance and the data previously read and indicated on the display unit 6, arithmetic operations are carried out by control unit 10 to calculate a body fat occupancy ratio. The results of the arithmetic operations and the previously read data are indicated on the display unit 6 (step 314). At the end of the display mode, data serving as the results of the display mode is stored in the memory 14. Depending on whether the Enter switch 5 is operated, it is judged whether the stored data will be used when the display mode is selected in the near future. If the data will not be used, control is passed to step 307. When the data will be used, the data is stored in the memory 14 at step 316. Control is then passed to step 307.

In the above display state, if the Mode switch 2 is operated, judgment is made in the negative at step 311, and the display of step 314 is deleted. Control is passed to the new measurement mode involving step 317 to step 329 for measuring a body fat occupancy ratio. At step 318, a sex of male or female is indicated on the display unit 6. The user's sex is designated by operating the Up switch 3 or Down switch 4. When the Enter switch 5 is operated, the data is finalized (step 319).

A message prompting the user to enter his/her age is indicated at step 320. In addition to the message, an initial value of an age to be entered, for example 20 years old, is indicated on the display screen used to enter the age. When the Up switch 3 or Down switch 4 is operated repeatedly or held down, the numerical value is increased or decreased in order to thus set the user's age.

When the Enter switch 5 is operated, the data is finalized (step 321). At step 322, a message prompting the user to enter his/her height is indicated. At step 323 to step 325, similarly to steps 320 and 321, the Up switch 3 and Down switch 4 are used to enter the user's height and weight. The data of the height and weight is finalized using the Enter switch 5. The processing of step 326 to step 329 is identical to that of step 313 to step 316, so a description of this processing will be omitted. The display state established at step 327 persists as the display state for the new measurement mode.

In the display state, when the Mode switch 2 is operated, judgment is made in the negative at step 317, the indication displayed at step 327 is deleted, and control is passed to the alarm setting mode involving step 330. In the alarm setting mode, a well-known alarm setting precedure, such as performed on a clock, is carried out. A description of this processing will therefore be omitted. When the alarm setting is completed, control is returned to step 304. The processing of step 304 to step 307 is repeated, whereby a current time is indicated. When the Mode switch 2 is operated consecutively a plurality of times, control jumps directly to a mode described in the flowchart of Fig. 3 according to the number of times by which the Mode switch 2 is operated.

According to the present embodiment, when the Mode switch 2 is operated three times, the alarm setting mode is established. When the Mode switch 2 is operated four times, the alarm setting mode is changed back to the current time display mode. When the Mode switch 2 is operated five times or more, the modes are changed forwards sequentially. Additionally, the inventive portable body fat monitor may be used by a plurality of users. In this case, a step of indicating data concerning a plurality of measurers and a step of selecting displayed data using the Up and Down switches 3, 4 and finalizing selected data using the Enter switch 5 are included as steps succeeding step 309, 311, or 317. Moreover, for storing data concerning a plurality of users in a memory, a step of selecting any of the plurality of users in relation to which data is stored may be included as a step succeeding step 315 or 328.

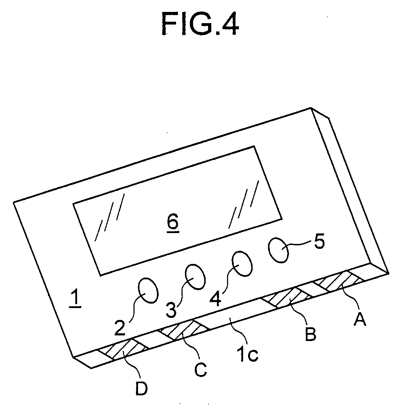

According to this embodiment of the present invention, four electrodes are formed on the upper side surface 1b of the main body 1. Alternatively, the electrodes may be formed on a lower side surface 1c thereof, as shown in Fig. 4. In this case, measurement can be carried out with the user's palms oriented upward. At this time, the index finger of the left hand is brought into contact with the electrode A, and the middle finger thereof is brought into contact with the electrode B. The middle finger of the right hand is brought into contact with the electrode C, and the index finger thereof is brought into contact with the electrode D. However, the fingers coming into contact with the electrodes are not limited to the above fingers but may be varied depending on how the main body is held.

In the aforesaid embodiments, the main body is shaped nearly like a parallelepiped. However, the shape is not limited to any specific shape as long as the main body can be held with the fingers of two hands. The shape may be, for example, an elliptic or circular cylinder.

The present invention can be practiced by employing conventional materials, methodology and equipment. Accordingly, the details of such materials, equipment and methodology are not set forth herein in detail. In the previous descriptions, numerous specific details are set forth, such as specific materials, structures, chemicals, processes, etc., in order to provide a thorough understanding of the present invention. However, it should be recognized that the present invention can be practiced without resorting to the details specifically set forth. In other instances, well known processing structures have not been described in detail, in order not to unnecessarily obscure the present invention.

Only the preferred embodiment of the present invention and but a few examples of its versatility are shown and described in the present disclosure. It is to be understood that the present invention is capable of use in various other combinations and environments and is capable of changes or modifications within the scope of the inventive concept as expressed herein.

## Claims

1. A portable body fat monitor comprising:
a body having a front face and a side surface;
four electrodes spaced apart from each other on the side surface;
a display unit on the front face;
an operator unit on the front face; and
a circuit contained within the body and coupled to the four electrodes, display unit and operator unit.

2. The monitor of claim 1, wherein the body is substantially shaped as a parallellepiped, a cylinder or an elliptic cylinder.

3. The monitor of claims 1 or 2, wherein the side surface is an upper side surface of the main body.

4. The monitor of claims 1 or 2, wherein the side surface is a lower side surface of the main body.

5. The monitor of one of claims 1 to 4, wherein the operator unit comprises a plurality of electrical switches.

6. The monitor of one of claims 1 to 5, wherein the body comprises an insulating material.

7. The monitor of one of claims 1 to 6, wherein the four electrodes are arranged in a row on the side surface.

8. The monitor of one of claims 1 to 7, wherein the four electrodes are spaced from each other such that four fingers of a user can simultaneously contact the four electrodes without touching one another.

9. The monitor of one of claims 1 to 8, wherein the monitor is for measuring a bioelectrical impedance and calculating a body fat occupancy ratio based on the bioelectrical impedance.

10. The monitor of claim 9, further comprising a processor for calculating the body fat occupancy ratio based on signals from the electrodes.

11. The monitor of one of claims 1 to 10, wherein the four electrodes are arranged as two pairs of electrodes, and the two pairs of electrodes are spaced apart from each other.

12. The monitor of claim 5, wherein the electrical switches comprise push buttons.

13. The monitor of claim 12, wherein the push buttons are disposed on the front face of the body for operation by a user's thumbs when the user is holding the body in the user's hands.

14. The monitor of claim 8, wherein two of the four fingers are of the user's right hand, and two of the four fingers are of the user's left hand, when the user is holding the body in the user's hands.

15. The monitor of one of claims 1 to 14, wherein the circuit comprises:
a constant current generating circuit connected to a first pair of the four electrodes;
a voltage measurement circuit connected to a second pair of said four electrodes;
a control unit; and
a memory.
